# EUROPEAN PATENT APPLICATION

(11) **EP 1 136 073 A1**
(43) Date of publication of application: **26.09.2001**
(21) Application number: 00201051.0
(22) Date of filing: 22.03.2000
(51) Int. Cl.: A61K 35/78, A61K 45/06, A61K 31/35, A61P 9/10

(54) **Compositions suitable for the treatment of damage caused by ischemia/reperfusion or oxidative stress**

(71) Applicant: N.V. Nutricia, 2700 MA Zoetermeer (NL)
(72) Inventor: Van Norren, Klaske, 6871 LP Renkum (NL); Van Hoorn, Eduard Christiaan, 6671 AZ Zetten (NL); Leuvenink, Hendrik Gerrit Derk, 9481 HN Vries (NL); Hofman, Zandrie, 6721 RH Bennekom (NL)
(74) Representative: Jorritsma, Ruurd

(57) **Abstract**

The present invention relates to a preparation containing
a) at least one substance having xanthine oxidase inhibiting properties;
b) at least one substance having superoxide radical scavenging properties;
c) at least one substance having peroxyl radical scavenging properties;
These preparations are particularly suitable for treating damage caused by ischemia/reperfusion and oxidative stress, preventing multiple organ failure, treating subfertility and treating or preventing peri-operative complications

## Description

The present invention relates to compositions containing substances which are able to repair or prevent damage caused by ischemia/reperfusion or oxidative stress. More in particular the present invention relates to a composition containing different substances each interfering at a different stage of the radical cascade caused by the above mentioned disorders.

Ischemia/reperfusion and oxidative stress related diseases and injuries are a widespread problem world wide. These processes can take place all over the body and are instantly occurring, very fast and cascade like. An approach of braking the cascade chain at only one side, carries the danger of highly reactive components slipping through, and because each component of the chain can start a reaction chain on its own, this implicates an inefficient way of approaching the problem.

The invention is based on the approach via a mixture of components, that together can interfere at three different stages of the radical cascade. In this way providing a more accurate and effective defence against ischemia/reperfusion and oxidative stress mediated injuries and diseases.

The cascade can be illustrated as follows:

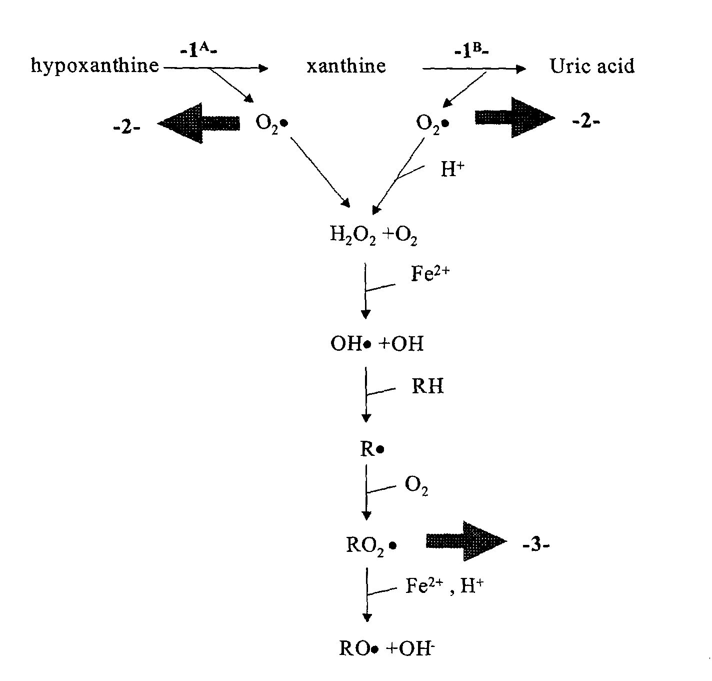

According to the present invention, there is provided a preparation containing
a) at least one substance having xanthine oxidase inhibiting properties;
b) at least one substance having superoxide radical scavenging properties;
c) at least one substance having peroxyl radical scavenging properties;

The properties of these substances a), b) and c) can be further defined:

Substance a) preferably has an IC-50 value of less than 10 µM as determined by the xanthine oxidase assay. In the xanthine oxidase assay the reduction of xanthine oxidase activity to convert xanthine to uric acid is measured.

Substance b) preferably has an IC-50 value of less than 10 µM as determined by the superoxide scavenging assay. In the superoxide scavenging assay the reduction of the concentration superoxide radicals resulting from conversion of hypoxanthine by xanthine oxidase is measured. Moreover, the IC-50 value of a substance b) in the superoxide scavenging assay is less than the IC-50 value of said substance in the xanthine oxidase assay in order to discriminate the anti-oxidant from the enzyme inhibitory activities and to mark the component as a superoxide radical scavenging component.

Substance c) has a value greater than 1.0 in relation to the value for trolox on a molar basis, as determined in the β-phycoerythrin fluorescence based assay. In the β-phycoerythrin fluorescence based assay the reduction of β-phycoerythrin decomposition by radicals resulting from the thermal decomposition of V50 is measured. The value obtained for the substance to be tested is compared, on a molar basis, to the value for Trolox.

The above mentioned tests are further illustrated in the examples.

According to the present invention substances a), b) and c) can be any substance, natural or synthetic, as long as they fulfil the above-described criteria. Examples of substance a) having xanthine oxidase inhibiting properties are quercetin, myricetin, luteolin, chrysin, apigenin and allopurinol.

Preferably polyphenols, more preferably flavonoids, are used, in particular those selected from the group consisting of compounds having formula (I) 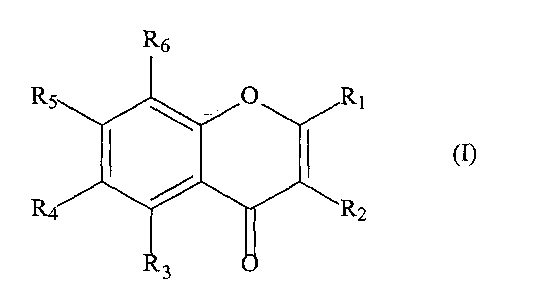 wherein

R₁ and R₂ are independently H, OH, O, Cl, Br, I, F, N or a group with formula (II) 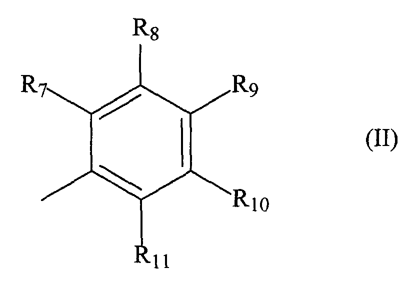 wherein R₇, R₈ R₉, R₁₀ and R₁₁ are independently OH, H, CH₃, OGluc Cl, Br, I, F or N; R₃, R₄, R₅ and R₆ are independently OH, H, CH₃, OGluc, Cl, Br, I, F or N; wherein Gluc stands for glucose.

According to a preferred embodiment of the present invention R₁ is a group with formula (II) and R₂ is OH or H;
R₈, R₉ and R₁₀ are OH or H and R₇ and R₁₁ are H; and
R₃ is OH, R₄ is H, R₅ is OH and R₆ is H or R₃ is H, R₄ is H, R₅ is OH and R₆ is OH, or R₃ is H, R₄ is OH, R₅ is H and R₆ is OH.

In particular substance a) is selected from apigenin, chrysin, luteolin, myricetin, morin and quercetin.

Substance b) having superoxide radical scavenging properties can be any natural or synthetic substance which complies with the above-mentioned criterion. Examples thereof are epicatechin, epigalocatechin, epigalocatechinochalate, taxifolin.
Preferably substance b) is selected from flavonoids. Most preferably substance b) is epicatechin or taxifolin.

Substance c) having peroxyl radical scavenging properties can be any natural or synthetic substance which complies with the above-mentioned criterion and is preferably selected from quercetin, epicatechin, luteolin.

All the above values in the assays are based on pure substances. However, mixtures of substances that comply with any of the three criteria can also be used. Most preferred mixtures are extracts of natural source which contain flavonoids. As a substance a) onion, hypericum perforatum, chammomillae, linden flowers, thyme, parsley extracts can be used. As a substance b) for instance green tea extract, Aerola or Hamamelidis extract can be used. As a substance c) for instance green tea extract or grape skin extract can be used.

In the preparation of the present invention the xanthine oxidase inhibiting components should be present in an amount such that at least x mg/day is administered, wherein: Σx/(IC-50^{*}D) = 49
wherein the IC-50 is determined according to the xanthine oxidase assay (µg/ml). D = 1000 ml/day. D is a correction factor for the formula, it is not related to the administration of the components. In case of luteolin this means that at least 10 mg/day luteolin is administered, when this is the only xanthine oxidase inhibitor present. For quercetin this is 25 mg/day.

The superoxide radical scavengers should be present in an amount such that at least x mg/day is administered, wherein:
Σx/(IC-50^{*}D) = 14
wherein the IC-50 is determined according to the superoxide assay (µg/ml). D = 1000 ml/day. D is a correction factor for the formula, it is not related to the administration of the components. In case of epicatechin this means that at least 9.8 mg/day epicatechin is administered, when this is the only superoxide scavenger present.

The peroxide scavenger components should be present in an amount such that at least x mg/day is administered, wherein:
x ^{*} trolox equivalent = 18.

The trolox equivalent is determined according to the β-phycoerythrin fluorescence based assay (mg). In case of apigenin this means that at least 10.9 mg/day apigenin is administered, when this is the only lipid peroxide scavenger present.

The maximum amount of the substance to be administered is not limited. In general at a certain level no additional effect will be observed because the transport of the substance into the blood of the patient will be limited. For economical reasons the maximum amount of the substance will be the amount at which no additional effect occurs. This can be determined by determining plasma concentrations and testing these concentrations in in vitro assays.

The present invention also relates to the use of the preparation according to the invention for the treatment of damage caused by ischemia/reperfusion. An ischemia/reperfusion mediated condition is herein defined as a condition caused by a reduced blood flow, or a reduced maximal blood flow to a certain organ, tissue or part of an organ or tissue; followed by a period of re-established blood flow. It is also possible that the concentration of oxygen is not matching the demands for certain time periods. This is for example the case in patients suffering from breathing problems as for example COPD, ARDS, and cystic fibrosis.

The preparation can also be used for the treatment of oxidative stress. Because xanthine oxidase can start the described cascade, events in which oxidative stress is associated with high levels of xanthine oxidase can be treated or prevented with the described preparation. In this case the prevention or treatment is more effective than with xanthine oxidase inhibitors alone because the invention warrants the stop of the cascade at three levels.

Examples of damage caused by ischemia/reperfusion and oxidative stress are multiple organ failure, subfertility, disorders that occur in relation to surgical procedures, damage to organs after surgical procedures, damage to lungs after damage to tissue or organs in other parts of the body, for instance lung damage after burn injuries or after pancreatitis, organ transplants, heart failure or angina, COPD or ARDS, cystic fibrosis, Inflammatory Bowel Disease (*1*, *7, 15*), circulatory shock, gout, rheumatoid arthritis (*10*, *12),* osteoarthrosis (*6*), protection against liver damage caused by use of medication or ethanol (*3*), inflammation and disorders related therewith, hypertension (*13*), muscle damage after sports (*4*), acute renal failure (*2*), pre-enclampsia or enclampsia (*8*, *9*), sepsis (*14*).

The application of the present preparation in surgery is important. For example, during surgery arteries can be clamped to avoid excessive blood loss. If this clamp is removed, tissues and organs are reperfused. Moreover, during surgery blood is retained from the intestinal organs. This means that surgery itself induces a mild ischemia/reperfusion cycle for the intestine. Moving and twisting of tissue and organs e.g. large intestine (for the duration of a surgery) can also lead to short and local ischemia and reperfusion events. Another example in which surgery leads to an ischemia/reperfusion event is when blood flow to an ischemic part of the body is restored by surgery, e.g. a bypass of a heart, or by pursing of artherosclerotic regions in the leg. Elevations of xanthine oxidase in combination with oxidative stress can also occur in injured human skeletal muscle (*4*) or hypertension (*13*).

Multiple organ failure sometimes occurs when ischemia of one organ leads to ischemia of another organ. The liver receives most of the blood from the intestine. This means that ischemia/reperfusion of the intestine leads to ischemia/reperfusion of the liver. In this way not only damage to the intestine occurs, but also damage to the liver occurs. An injured liver releases all kinds of stored enzymes. One of these enzymes is xanthine oxidase (XO). The blood as drained from the liver by the vena cava reaches the lung. The lung is oxygen rich and therefore oxygen radicals can easily be formed. The xanthine oxidase produced by the liver can induce damage in the lung. Similar cascades can happen toward kidney and from lung to liver. Hereafter preliminary data are given in which components mixed according to the invention are given to rats in order to prevent this multiple organ failure cascade.

Another specific example where ischemia/reperfusion causes damage is organ transplantation. Organs are removed from the donor (ischemic period) and transplanted into the recipient (reperfusion period). This means that the invention can be used for preparations given to the recipient to decrease ischemia/reperfusion effects in the body, as well as for fluids to preserve the organs before being transplanted into the recipient.

The preparation can also be used in case of subfertility. Oxidative stress in combination with high xanthine oxidase levels can be observed in subfertility in men (*5*, *11*). The present invention will reduce XO activity and enhance radical scavenging and therefore improve fertility.

Another example of restoration of blood flow takes place after a circulatory shock or after a severe blood loss.

Ischemia/reperfusion also takes place in rheumatoid arthritis and osteoarthrosis. During the swelling of the joints reduction in blood flow to the joint due to the pressure of the swelling can occur. This leads to ischemia. Reduction of the swelling leads to perfusion (*6*, *10*).

The invention is illustrated by the examples, which also show the assays used to determine the properties of the different substances used.

### Examples

### Determination of properties

### Xanthine oxidase inhibition (xanthine oxidase assay)

The activity of xanthine oxidase is determined by means of the uric acid production. Materials used:
phosphate buffer: 50 mM Na₂HPO₄; MW = 141.96 g/mol; 7.10 g/l, pH adjusted to 7.8 with 1 M HCl
xanthine solution: 21.2 mg/250 ml phosphate buffer
(for dissolving the xanthine this mixture is agitated ultrasonically for 1,5 hours and heated to 90 °C for 5 to 10 minutes)
enzyme solution: XOD (xanthine oxidase) (grade I from buttermilk) 18.5 U/ml, 10.2 µl XOD 18.5 U/ml in 5000 µl in ice-cold phosphate buffer (189^{*}10⁻³ U/5000 µl). This solution is maintained on ice during the entire experiment to prevent changes in the activity of the enzyme.

### Method:

Assay mixture:
724 µl phophate buffer pH=7.8
200 µl xanthine solution
10 µl DMSO or the substance to be tested dissolved in DMSO

The reaction is started by addition of 66 µl of the enzyme solution to the above described assay mixture. The reaction is monitored after half a minute from the start for 6 minutes, by measuring the absorbance at 295 nm. If the reaction is linear, the result is expressed as Δabsorbance/min. The absorbance at 295 nm is a measure for the uric acid concentration.

The IC-50 value is determined as the concentration of the substance to be tested corresponding to 50 % inhibition as compared to the maximum activity. The maximum activity is the activity of the enzyme, when no substance is dissolved in DMSO.

### Examples of substances and extracts having xanthine oxidase inhibiting properties:

| Sample | IC-50 (µg/ml) | IC-50 (µM) |
|---|---|---|
| Luteolin | 0.215 | 0.75 |
| Apigenin | 0.27 | 1.0 |
| Myricetin | 0.48 | 1.5 |
| Quercetin dihydrate | 0.51 | 1.5 |
| Chrysin | 0.645 | 2.5 |
| Allopurinol | 0.84 | 6.2 |
| Catechin | not detectable | not detectable |
| Epicatechin | not detectable | not detectable |
| Curcumin | not detectable | not detectable |
| Epigalo-catechin-galate (ECCG) | not detectable | not detectable |

These materials were obtained from Indofine chemicals and Sigma.

### Superoxide radical scavenging (superoxide scavenging assay)

Literature reference: Re-evalutation of assay methods and establishment of kit for superoxide dismutase activity, Yoshihiko Oyanagui, Analytical Biochemistry 142, 290-96 (1984).

### Materials used:

All solutions are prepared with analytical grade chemicals and milliQ water.
Buffer A: 0.2 mM hydroxylamine and 1.87 mM hypoxanthine dissolved in buffer B. For dissolving the components it can be necessary to ultrasonically agitate the mixture of the substances followed by heating the mixture to 80 °C for several minutes.
Buffer B: 20.8 mM K₂HPO₄ and 15.6 mM Na₂B₄O₇, the pH being adjusted to 7.0 with 1 M NaOH
Buffer C (colouring reagent): 300 µg/ml sulfanilic acid and 10 µg/ml N-(1-naphtylethylenediamine) dissolved in 16.7 % acetic acid.
Enzyme solution: xanthine oxidase from Buttermilk Grade I 5.25 µl of 18.5 U/ml dissolved in 7575 µl buffer B (97^{*}10⁻³ U in 7575 µl).

### Method:

10 µl of the substances to be tested were dissolved in DMSO or milli Q water and added to and mixed with, respectively: 390 µl buffer B and 200 µl buffer A. Next 400 µl of the enzyme solution is added and mixed and the sample is placed directly at 37 °C and incubated at this temperature for 30 minutes. Than the reaction is stopped by adding 2 ml of buffer C. The sample is then incubated at 20 °C for 20 minutes and the absorbance at 550 nm is measured. The absorbance is a result of reaction of a coloring reagent with remaining superoxide radicals and therefore a measure of the amount of superoxide radicals remaining.

The results were compared with a reference containing only the solvent, i.e. either DMSO or milliQ water.

The IC-50 value is the concentration of a substance or mixture of which the scavenging activity is 50% of the maximum activity. The maximum activity is the absorbance at 550 nm of the above mentioned reference.

The absorbance measured is corrected for background colouring. The correction factor is the absorbance at 550 nm of a sample, incubated as described above, but containing only 10 µl of DMSO and to which instead of 400 µl enzyme solution, 400 µl buffer B is added.

### Examples of substances having superoxide scavenging properties

| Sample | IC-50 (µg/ml) | IC-50 (µM) |
|---|---|---|
| Epicatechin | 0.7 | 2.4 |
| Myricetin | 1.6 | 3.4¹⁾ |
| Taxifolin | 2.8 | 9.2 |
| QDH | 3.6 | 10.8¹⁾ |
| Luteolin | | >100 |
| Apigenin | | >100 |
| Chrysin | | >100 |
| Catechin | | >100 |
| curcumin | | >100 |
| ECCG | | >100 |
| Vitamin C | 4.4 | 25 |

| | | |
|---|---|---|
| ¹⁾ These substances are excluded because the IC-50 or superoxide radical scavenging » IC-50 xanthine oxidase inhibition. | | |

### Peroxyl radical scavenging (β-phycoerythrin fluorescence assay)

### Literature reference:

1.Oxygen Radical Absorbance capacity assay for antioxidants, Guohua Cao et al, Free Radical Biology and medicine, vol. 14, pp. 303-311, 1993.

2. Phycoerythrin fluorescence based assay for peroxyl radicals: A screen for biologically relevant protective agents; Delange, R.J., Glazer A.N., Anal. Biochem., 177; 300-306 (1989).

In order to avoid differences in result by small changes in measuring conditions, such as analytical instruments, sensitivity, etc. the end results are expressed in relation to the peroxyl scavenging capacity of a known amount of Trolox (a vitamin E analogue).

The principle underlying the present method is that B-phycoerythrin is decomposed by radicals that are the result of the thermolitic decomposition of V50 at 20 °C. When a product scavenges these radicals, the decrease in fluorescence in time will occur less quickly. All measurements should be carried out at least in threefold.

### Materials used:

Equipment: Fluorimeter for 96 wells. The temperature of the tray must be kept constant. The variation between the highest and the lowest temperature of the wells should not be more than 0.5 °C. 96 well white trays of Perkin and Elmer were used.

### Chemicals:

AAPH (2,2'-azobis(2-aminopropane)dihydrochloride) (e.g. from Wako Chemicals)= V50; MW = 271.19 g/mol; 16 mM in the assay
β-PE (e.g. from Sigma) MW=240,000 g /mol; 16.7 nM in the assay

Trolox (e.g. from Aldrich) MW = 250.29 g/mol; in the assay test in the range of 1-10 µM.
Conditions in the assay ORAC_{ROO}:
10 µl sample in ethanol
50 µl 50 mM PBS pH=7.4
50 µl 66.8 nM β-PE
Each tray should contain trolox as a reference and as a standard (2 µM or 0.5 mg/l). As a negative control a sample is taken containing only 10 µl ethanol, 50 µl 50 mM PBS, pH=7.4 and 50 µl 66.8 nM β-PE.

The reaction is started by adding 100 µl 32nM V-50. The time between pipetting and mixing of this last step in a well and the measurement of this well, should be constant. Further, the time between the last pipetting step in a well and the first measurement of the well should be less than 2 minutes.

V-50 should be stored in the dark at 4 °C until used. The reaction kinetics of the wells are monitored in a continuous monitoring process. The excitation wavelength is 540 nm, while the emission wavelength is 565 nm. The reaction is monitored until the signal of the sample differs no longer from the background signal (+/- 10%). The area underneath the curve of the negative reference is subtracted from the area underneath the substance to be measured. This difference in area is then related to a difference in area in relation to a negative reference resulting from trolox.

### Examples

| Sample | TE (Trolox equivalent) (based on mass) | TE (Trolox equivalent) (based on mole weight) |
|---|---|---|
| apigenin | 1.56 | 1.69 |
| quercetin | 1.32 | 1.78 |
| curcumin | 1.27 | 1.72 |
| epicatechin | 1.24 | 1.44 |
| chrysin | 1.23 | 1.25 |
| luteolin | 1.22 | 1.40 |
| fustin | 1.07 | 1.24 |
| myricetin | 0.89 | 1.13 |
| EGCG | 0.54 | 0.99 |

A mixture having xanthine oxidase inhibiting, superoxide radical scavenging and peroxyl radical scavenging properties according to the present invention was used in an in vitro experiment to show that this mixtures indeed had these properties. Next a rat model was developed in which a cascade of multiple organ failure occurs. In this model rats were fed a normal feed or a feed of a flavonoid mixture according to the present invention.

An experiment was carried out in male Wistar rats. Rats were fed for 5 days a flavonoid enriched diet containing luteolin, quercetin, apigenin, epicatechin and tea extract, or a control diet. Rats were not fasted, but could eat ad libitum until the anaesthesia. Rats were anaesthetised and a clamp on the arteria mesenterica was placed. The experiment was carried out in two groups of nine rats. Ischemia was performed for one hour, followed by two hours of reperfusion. Biochemical parameters were measured in order to check if xanthine oxidase had been inhibited. Parameters indicating oxidative stress and other parameters indicating damage to an organ were measured.

### 1. Biochemical

Determination of uric acid and allatonin levels.

| | Comparative | SE | Flavonoid mix | SE | p |
|---|---|---|---|---|---|
| Uric acid (µmol/l) | 386 | 193.5 | 261 | 90.9 | 0.03 |

Xanthine oxidase was significantly inhibited by the flavonoid mix. As is shown in the above table uric acid levels decrease significantly, when flavonoids are added.

### 2. Small intestine

| | Comparative | SE | Flavonoid mix | SE | p |
|---|---|---|---|---|---|
| Apoptose | 2.58 | 1.91 | 0.20 | 0.11 | 0.02 |

A significant reduction was found in apoptose (spontaneous cell death) in animals fed with the flavonoid mixture.

### 3. Liver parameters

| | Comparative | SE | Flavonoid mix | SE | p |
|---|---|---|---|---|---|
| Plasma: | | | | | |
| ALAT (U/L) | 47 | 4.1 | 45.8 | 7.3 | 0.45 |
| Arginase (µmol/min) | 2.35 | 0.55 | 1.83 | 0.71 | 0.28 |
| Bilburine (µmol/L) | 1.7 | 0.6 | 1 | 0 | 0.18 |
| ASAT (U/L) | 94.3 | 13.3 | 78 | 9.5 | 0.15 |
| GGT (U/L) | 0.4 | 0.2 | 0.1 | 0.1 | 0.12 |
| Urea (mmol/L) | 8.8 | 3.4 | 7.2 | 0.6 | 0.11 |
| Tissue homogenates | | | | | |
| 8-iso-PGF2 alpha (pg/ml) | 304.7 | 36 | 242.2 | 30 | 0.12 |
| MDA (nM) | 263.6 | 12.1 | 236.7 | 13.1 | 0.075 |

All parameters described above are parameters indicating liver injury. From the table above tendency towards liver injury can be detected.

### 4. Kidney function

| | Comparative | SE | Flavonoid mix | SE | p |
|---|---|---|---|---|---|
| Creatinine (µmol/l) | 57.4 | 7.1 | 49.4 | 2.9 | 0.16 |

Also kidney function and in general lactate production seem to be decreased when flavonoids are administered.

### 5. General

| | Comparative | SE | Flavonoid mix | SE | p |
|---|---|---|---|---|---|
| Lactate (mmol/L) | 1.9 | 0.2 | 1.6 | 0.1 | 0.2 |

In general: intestine function is improved significantly and also other organs seem to profit from the invention.

### References:

1. Buffinton, G. D. & Doe, W. F. Depleted mucosal antioxidant defences in inflammatory bowel disease. Free Radic Biol Med 19, 911-8 (1995).
2. Edelstein, C. L., Ling, H. & Schrier, R. W. The nature of renal cell injury. Kidney
Int 51, 1341-51 (1997).
3. Grattagliano, I., Vendemiale, G., Sabb, C., Buonamico, P. & Altomare, E. Oxidation of circulating proteins in alcoholics: role of acetaldehyde and xanthine oxidase. J Hepatol 25, 28-36 (1996).
4. Hellsten, Y., Frandsen, U., Orthenblad, N., Sjádin, B. & Richter, E. A. Xanthine oxidase in human skeletal muscle following eccentric exercise: a role in inflammation. J Physiol (Lond) 498, 239-48 (1997).
5. Kurpisz, M., Miesel, R., Sanocka, D. & Jedrzejczak, P. Seminal plasma can be a predictive factor for male infertility. Hum Reprod 11, 1223-6 (1996).
6. Liang, H. J., Tsai, C. L., Chen, P. Q. & Lu, F. J. Oxidative injury induced by synthetic humic acid polymer and monomer in cultured rabbit articular chondrocytes. Life Sci 65, 1163-73 (1999).
7. Lih Brody, L. et al. Increased oxidative stress and decreased antioxidant defenses in mucosa of inflammatory bowel disease. Dig Dis Sci 41, 2078-86 (1996).
8. Many, A. & Roberts, J. M. Increased xanthine oxidase during labour--implications for oxidative stress. Placenta 18, 725-6 (1997).
9. McKenzie, S. J., Baker, M. S., Buffinton, G. D. & Doe, W. F. Evidence of oxidant-induced injury to epithelial cells during inflammatory bowel disease. J Clin Invest 98, 136-41 (1996).
10. Milam, S. B., Zardeneta, G. & Schmitz, J. P. Oxidative stress and degenerative temporomandibular joint disease: a proposed hypothesis. J Oral Maxillofac Surg 56, 214-23 (1998).
11. Sanocka, D., Miesel, R., Jedrzejczak, P. & Kurpisz, M. K. Oxidative stress and male infertility. J Androl 17, 449-54 (1996).
12. Situnayake, R. D. et al. Chain breaking antioxidant status in rheumatoid arthritis: clinical and laboratory correlates. Ann Rheum Dis 50, 81-6 (1991).
13. Suzuki, H. et al. Xanthine oxidase activity associated with arterial blood pressure in spontaneously hypertensive rats. Proc-Natl-Acad-Sci-U-S-A. 95, 4754-9 (1998).
14. Taylor, D. E. & Piantadosi, C. A. Oxidative metabolism in sepsis and sepsis syndrome. J Crit Care 10, 122-35 (1995).
15. Thomson, A., Hemphill, D. & Jeejeebhoy, K. N. Oxidative stress and antioxidants in intestinal disease. Dig Dis 16, 152-8 (1998).

## Claims

1. Preparation containing
a) at least one substance having xanthine oxidase inhibiting properties;
b) at least one substance having superoxide radical scavenging properties;
c) at least one substance having peroxyl radical scavenging properties;

2. Preparation according to claim 1, wherein
substance a) has an IC-50 value of less than 10 µM as determined by the xanthine oxidase assay;
substance b) has an IC-50 value of less than 10 µM as determined by the superoxide scavenging assay; with the proviso that the IC-50 value of substance b) in the superoxide scavenging assay is less that the IC-50 value of said substance in the xanthine oxidase assay;
substance c) has a value greater than 1.0 in relation to the value for trolox on a molar basis, as determined in the β-phycoerythrin fluoresence based assay.

3. Preparation according to claim 1 or 2, wherein substance a) is selected from flavonoids, extracts of onion, hypericum perforatum, chammomillae, linden flowers, thyme and parsley, and allopurinol.

4. Preparation according to claim 2 or 3, wherein substance a) is selected from compounds having the formula (I) 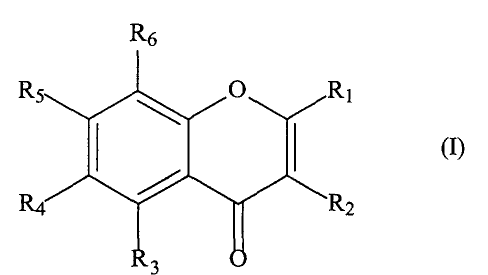 wherein
R₁ and R₂ are independently H, OH, O, Cl, Br, I, F, N or a group with formula (II) 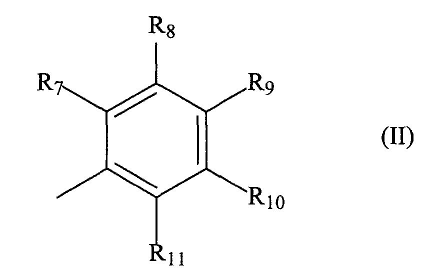 wherein R₇, R₈ R₉, R₁₀ and R₁₁ are independently OH, H, CH₃, OGluc Cl, Br, I, F or N; R₃, R₄, R₅ and R₆ are independently OH, H, CH₃, OGluc, Cl, Br, I, F or N.

5. Preparation according to claim 4, wherein in formula (I) R₁ is a group with formula (II) and R₂ is OH or H;
and in formula (II) R₈, R₉ and R₁₀ are OH or H and R₇ and R₁₁ are H; and
R₃ is OH, R₄ is H, R₅ is OH and R₆ is H or R₃ is H, R₄ is H, R₅ is OH and R₆ is OH, or R₃ is H, R₄ is OH, R₅ is H and R₆ is OH.

6. Preparation according to claim 5, wherein substance a) is selected from apigenin, chrysin, luteolin, myricetin, morin and quercetin.

7. Preparation according to any of claims 1 to 6, wherein substance b) is selected from flavonoids.

8. Preparation according to claim 7, wherein substance b) is selected from epicatechin, taxifolin or green tea extract.

9. Preparation according to any of claims 1 to 8, wherein substance c) is selected from quercetin, epicatechin, luteolin and green tea extract.

10. Preparation according to claim 9, wherein substance c) is selected from green tea extract.

11. Use of a preparation according to any of claims 1 to 10 for the preparation of a medicament for the treatment of damage caused by ischemia/reperfusion and oxidative stress.

12. Use according to claim 11, to prevent multiple organ failure

13. Use according to claim 11, to treat subfertility.

14. Use according to claim 11, to treat or prevent peri-operative complications.
